# EUROPEAN PATENT SPECIFICATION

(11) **EP 4 322 829 B1**
(45) Date of publication and mention of the grant of the patent: **29.07.2026**
(21) Application number: 21937153.1
(22) Date of filing: 14.04.2021
(51) Int. Cl.: A61B 5/00, A61B 5/026, A61B 5/145, A61B 5/1473

(54) **TISSUE PERFUSION SENSOR AND PLACEMENT DEVICE**
GEWEBEPERFUSIONSSENSOR UND PLATZIERUNGSVORRICHTUNG
CAPTEUR DE PERFUSION DE TISSU ET DISPOSITIF DE PLACEMENT

(43) Date of publication of application: 21.02.2024
(73) Proprietor: Exostat Medical, Inc., Prior Lake MN 55372 (US)
(72) Inventor: PIERSKALLA, Irvin, T., Prior Lake, MN 55372 (US); WINGER, Kent, R., Prior Lake, MN 55372 (US)
(74) Representative: Reddie & Grose LLP
(86) International application number: PCT/US2021/027261
(87) International publication number: WO 2022/220823

(56) References cited:
- WO-A1-2021/076194
- US-A- 6 055 447
- US-A1- 2002 087 057
- US-A1- 2003 225 324
- US-A1- 2008 319 278
- US-A1- 2008 319 278
- US-A1- 2010 044 226
- US-A1- 2012 271 131
- US-B2- 11 013 434
- US-B2- 8 062 221
- LU Z ET AL: "A high precision, fast response, and low power consumption in situ optical fiber chemical pCO"2 sensor", TALANTA, ELSEVIER, AMSTERDAM, NL, vol. 76, no. 2, 15 July 2008 (2008-07-15), pages 353 - 359, XP022686066, ISSN: 0039-9140, [retrieved on 20080315], DOI: 10.1016/J.TALANTA.2008.03.005

## Description

### CROSS REFERENCE TO RELATED APPLICATION(S)

This application claims benefit to U.S. Application Ser. No. 17/230,020 filed April 14, 2021. .

### TECHNICAL FIELD

The disclosure relates to the field of sensors for measuring the partial pressure of carbon dioxide (pCO₂) in tissue. More particularly, the disclosure relates to sensors for measuring the partial pressure of carbon dioxide in mucosal tissue.

### BACKGROUND

Very low blood flow, known as hypoperfusion can be caused by low blood volume, inadequate pumping action of the heart, or excessive widening (dilation) of blood vessels.

The body responds to such stress by reducing blood flow to less critical organs, such as the gastrointestinal tract, to spare blood for other, more critical organs. Thus, when there is a reduced flow of blood from the heart, the body directs a higher portion of blood to critical organs, such as the brain, which will not survive long without a continuous supply of blood, while restricting the flow of blood to less critical organs, whose survival is not as threatened by a temporary large reduction in blood flow.

For example, blood flow to the splanchnic vasculature, which supplies the stomach and intestines, and blood flow to the esophagus and oral/nasal cavity, is drastically reduced when there is reduced blood flow from the heart. For this reason, decreased blood flow to the splanchnic blood vessels is an indication of hypoperfusion in a patient. When hypoperfusion compromises intestinal mucosa, ischemia and gastric hypercapnia follow. These two clinical states can spur the release of bacteria and inflammatory substances into the splanchnic circulation, leading to sepsis and multiple organ dysfunction syndrome.

Carbon dioxide production, which is associated with metabolism, continues in tissues even during conditions of low blood flow. The concentration of carbon dioxide builds-up in tissues experiencing low blood flow because carbon dioxide is not rapidly carried away. This carbon dioxide build-up is exhibited by an increase in pCO₂ in organs. Therefore, hypoperfusion is commonly assessed by measuring pCO₂ at these sites.

Increases in pCO₂ may be measured throughout the body. Particularly, studies have shown that oral mucosal pCO₂ correlates well with gastric pCO₂ and thus oral mucosal constitutes an ideal site to measure pCO₂, especially if the sensing probe is isolated from ambient air and can be seated in a patient's mouth with minimal discomfort. Numerous studies have documented that both sublingual and buccal mucosal pCO₂ levels track circulatory stress in a quantitative fashion.

Measurements of pCO₂ have traditionally been taken with sensors having silicone membranes. Silicone membranes are useful because the large free volume in the polymer chain allows for rapid gas transport. Disadvantageously, however, silicone membranes also allow carboxylic acids, such as acetic acid, and other compounds found in saliva to pass through as well, which can interfere with pCO₂ measurements. For example, when acetic acid crosses the membrane into the sensor fluid, the pH is lowered and the conductivity of the fluid increases. Both alterations may falsely indicate an increase in carbon dioxide.

In addition, measurement of partial pressure of gases in tissue requires a sensor/tissue interface that is isolated from surrounding ambient air without application of excessive pressure. This has been attempted several ways, all which have limitations. First, measurement on the external epidermis has used adhesive patches and gels to isolate and capture the gas environment. This method is not practical for use on oral mucosal tissue which is inherently moist.

Secondly, handheld devices have been employed sublingually, with the tongue helping to seal off ambient exposure. This method is prone to errors because of the user dependent nature as well as not being practical for extended application. Furthermore, a method has been proposed by Anderson (U.S. Pat. No. 8,996,090) wherein the device is constructed with a material that deforms in response to pressure. The Anderson method is dependent not only on the material selection, but also the design of the applicator. Achieving the correct amount of flexibility so as not to create excessive pressure but still have enough pressure to keep the sensor in contact is problematic over the range of buccal tissue thickness found in adult patients. Normal buccal tissue thicknesses can range from about 7 mm to 20 mm. Maintaining contact without disturbing microcapillary blood flow requires pressure of no more than 25 mm Hg. Pressure in excess of 25 mm Hg can cause occlusion of blood flow that can cause errors in the measurement and damage to tissue.

Therefore, what is needed is a new design that allows for the rapid transmission of carbon dioxide while preventing the transmission of low molecular weight acids found in the saliva. What is also needed is a tissue placement device that is designed to hold and position the sensor against tissue, such as mucosal tissue.
US 2008/0319278 A1 discloses a physiological sensing device comprises, in combination a sensor (4) for the measurement of the partial pressure of carbon dioxide (pCO2), a body temperature sensor (5) and a heart rate and oxygen saturation sensor (54). The sensor device can be used to continuously monitor the vital signs of a patient.
XP 22686066 A discloses A sensor system suitable for monitoring changes in partial pressure of carbon dioxide (pCO2) in surface seawater or in the atmosphere has been developed. Surface seawater samples are pumped into a PVC tube enclosing an inner Teflon AF tube, which served as a long pathlength gas-permeable liquid-core waveguide for spectrophotometry. The Teflon cell contains a pH-sensitive indicator-buffer solution consisting of bromothymol blue (BTB) and sodium carbonate. Carbon dioxide in the sample diffuses into the indicator-buffer solution to reach equilibrium, resulting in pH changes, which are detected by changes in the absorbance of BTB at wavelengths of 620 and 434 nm. The pCO2 in the sample is then derived from the pH change. The sensor has a response time of 2 min at the 95% equilibrium value and a measurement precision of 0.26-0.37% in the range 200-800 µatm pCO2. This chemical sensor takes advantage of a combination of long pathlength, multiple wavelength detection, indicator solution renewal, and in situ automatic control technology, and has the feature of low power consumption (the average being -4 W with a peak of -8W).
US 2012/0271131 A1 discloses a sensor for physiological constituent detection may be adapted to include a mucoadhesive. A sensor is provided that is appropriate for use on mucosal tissue. The mucoadhesive provides a mechanism for holding the sensor on the mucous membrane in order to measure physiological constituent levels in the tissue and blood.
US 2010/0044226 A1 discloses a carbon dioxide sensor and a method of detecting carbon dioxide using the sensor are provided, the sensor includes a closed chamber having as a wall portion thereof a substantially watertight, carbon dioxide-permeable membrane, two electrodes disposed in the chamber, and a film of substantially electrolyte-free liquid disposed in the chamber capable of simultaneously contacting the membrane and both of the electrodes.
WO 2021/076194 A1 discloses A physiologic sensor for measuring the partial pressure of carbon dioxide is provided. The sensor includes a generally C- shaped in cross-section sensor cover, the sensor cover defining an opening on an underside thereof; a membrane body housed within the opening, the membrane comprising an amorphous fluoroplastic, the membrane including a first end and a second end and defines a chamber therewithin; a sensor body for coupling the membrane to the sensor cover; two or more electrodes positioned within the membrane chamber; and a substantially electrolyte-free liquid contained within the membrane chamber and in contact with the two or more electrodes.
US 2003/0225324 A1 provides a device for contacting a surface of a tissue within a patient's body to determine a physiologic parameter of the patient. The device typically comprises a sensor responsive to the physiologic parameter and a probe housing the sensor. The probe is constructed to allow the sensor to be secured at a sensing area adjacent to a surface of a patient's tissue, without need for an adhesive and without disturbing the blood flow within the measurement region of the tissue. The device may also include a means for reducing interference in the sensing area. Preferably, the device further comprises an indicating means operably connected to the sensor for indicating an analyte quantity and/or concentration associated with the physiologic parameter.

### BRIEF SUMMARY

The foregoing problems are addressed by the carbon dioxide sensor and tissue placement device in accordance with the disclosure. The invention is defined in the appended claims.

### BRIEF DESCRIPTION OF THE DRAWINGS

For a better understanding of the disclosure, and to show how the same may be carried into effect, reference will now be made, by way of example, to the accompanying drawings, in which:
FIG. 1 is a side view of the carbon dioxide sensor in accordance with the disclosure.
FIG. 2 is a perspective view of the carbon dioxide sensor in accordance with the disclosure.
FIG. 3 is a bottom view of the carbon dioxide sensor in accordance with the disclosure.
FIG. 4 is a cross-sectional view of the carbon dioxide sensor in accordance with the disclosure taken along line A-A of FIG. 3.
FIG. 5 is a side view of a sensor placement device for positioning the carbon dioxide sensor against a buccal surface.
FIG. 6 is a perspective view of the sensor placement device for positioning the carbon dioxide sensor against a buccal surface.
FIG. 7 is a perspective view of the sensor placement device being used on a patient.
FIG. 8 is a graph illustrating a comparison of various membrane materials exposure to acetic acid.
FIG. 9 is a graph illustrating a comparison of silicone and amorphous fluoroplastic membranes in tissue.
FIG. 10 is a side view of the sensor placement device in accordance with the disclosure.
FIG. 11 is a bottom view of the sensor placement device in accordance with the disclosure.
FIG. 12 is a perspective view of the sensor placement device in accordance with the disclosure.
FIG. 13 is a side view of an alternative ratcheting version of the sensor placement device in accordance with the disclosure set to a 5mm separation.
FIG. 14 is a partial view of an alternative ratcheting version of the sensor placement device in accordance with the disclosure set to a 5mm separation.
FIG. 15 is a side view of an alternative ratcheting version of the sensor placement device in accordance with the disclosure set to a 10mm separation.
FIG. 16 is a partial view of an alternative ratcheting version of the sensor placement device in accordance with the disclosure set to a 10mm separation.

### DETAILED DESCRIPTION

In the drawings, like reference numerals designate identical or corresponding parts throughout the several views.

As used herein, the words "a," "an" and the like generally carry a meaning of "one or more," unless stated otherwise. The term "plurality", as used herein, is defined as two or more than two. The term "another", as used herein, is defined as at least a second or more. The terms "including" and/or "having", as used herein, are defined as comprising (i.e., open language).

Reference throughout this document to "one embodiment", "certain embodiments", "an embodiment", "an implementation", "an example" or similar terms means that a particular feature, structure, or characteristic described in connection with the example is included in at least one example of the present disclosure. Thus, the appearances of such phrases or in various places throughout this specification are not necessarily all referring to the same example. Furthermore, the particular features, structures, or characteristics may be combined in any suitable manner in one or more examples without limitation.

The term "or" as used herein is to be interpreted as an inclusive or meaning any one or any combination. Therefore, "A, B or C" means "any of the following: A; B; C; A and B; A and C; B and C; A, B and C". An exception to this definition will occur only when a combination of elements, functions, steps or acts are in some way inherently mutually exclusive.

Further, in individual figures, some components/features shown are drawn to scale to exemplify a particular implementation while other components and features are not drawn to scale.

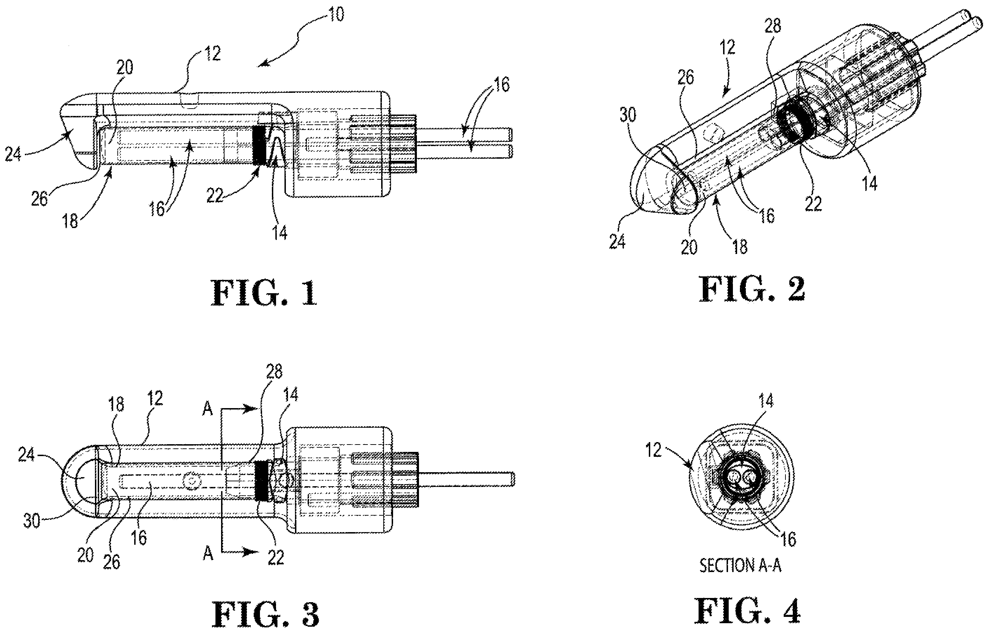

Referring now to FIG. 1 a side view of the carbon dioxide sensor in accordance with the disclosure is shown. The sensor 10 broadly includes sensor cover 12, sensor body 14, pair of electrodes 16, membrane body 18, sensor fluid 20 and winding filament 22.

The sensor cover may be constructed of a thermoplastic such as polyethylene, polypropylene, polystyrene and polycarbonate. The sensor cover 12 generally covers and houses the sensor body 14 and membrane body 18. Sensor cover 12 is shaped such that it forms an opening 26 on an underside thereof for housing the sensor body 14 and membrane body 18. One such shape is a generally C-shaped in cross section. Sensor cover 12 includes a downwardly projecting lip 24 that shields the membrane body 18 from end tidal carbon dioxide when in use. Advantageously, the sensor cover 12 permits tissue contact for greater than 40% to 50% of membrane body 18. The sensor cover 12 is designed to cover the portion of the membrane body 18 that is not in contact with tissue. Because the sensor placement device 58 is designed to fold the tissue around one side of the sensor, a cylindrical shape membrane body 18 is used. To cover the portion of the membrane body 18 that is not in contact with tissue, the inside surface of the sensor cover 12 may have a C-shaped cross section in order to minimize any gaps (dead-space that acts as a sink that can slow down the response of the sensor). In addition, the sides of the sensor cover 12 may be tapered to allow for better tissue contact.

Sensor body 14 may also be constructed of a thermoplastic such as polyethylene, polypropylene, polystyrene and polycarbonate. Sensor body 14 may be constructed of the same thermoplastic as sensor cover 12 or may comprise a different thermoplastic. Preferably, for manufacturing cost efficiencies sensor body 14 is constructed of the same thermoplastic as sensor cover 12. Sensor body 14 is configured to hold and align electrodes 16 securely in place within membrane body 18. Sensor body 14 provides an attachment point for membrane body 18 and for securing the winding filament 22 to provide a secure attachment between the sensor body and the membrane body 18. Those of skill in the art will appreciate that other attachments may be used such as snap-on, adhesives, bonding and crimping.

Pair of electrodes 16 are constructed of stainless steel and are configured to receive an alternating electrical potential from a supply source. Those of skill in the art will appreciate that metals other than stainless steel may also be used. Electrodes 16 are positioned securely in place by sensor body 14. Electrodes 16 are housed within membrane body 18 and positioned in sensor fluid 20. Two or more electrodes are used. For example, conductance can be measured with two, three, or four electrodes.

Membrane body 18 is positioned in opening 26 of sensor cover 12. Membrane body 18 comprises a hollow tube defining a chamber therewithin. The membrane body 18 is substantially impermeable to low molecular weight carboxylic acids, including acetic acid, which is found in salvia and can compromise precise readings of carbon dioxide levels in oral mucosa. Membrane body 18 is constructed of an amorphous fluoroplastic which is a fluoropolymer resin. Suitable amorphous fluoroplastics include Teflon AF 2400 (available from The Chemours Company). Teflon AF 2400 is known to have exceptional permeability for carbon dioxide. However, heretofore, it has been undiscovered that amorphous fluoroplastics, such as Teflon AF 2400, have a structure with a large free volume in the polymer chain that allows for rapid carbon dioxide transport but also does not permit carboxylic acids, such as acetic acid, to transport across it. Teflon AF 2400 has a carbon dioxide permeability of 2800 Barrer units as compared to polytetrafluoroethylene which has a carbon dioxide permeability of 120 Barrer units. Alternatively, polymethylpentenes (available from Mitsui Chemicals America) may be used in place of an amorphous fluoroplastic (however this alternative is not part of the claimed invention). Membrane body 18 is open at a first end 28 to allow for filling with sensor fluid 20 prior to attachment to the sensor body 14, which then seals it. A second end 30 is sealed with Teflon AF 1600, which has a much lower carbon dioxide transmission rate than AF 2400. Teflon AF 1600 easily fuses to membrane body 18 and provides a leak free environment. The second end of the tube is situated against lip 24 so it does not contact tissue and does not need to be permeable to carbon dioxide. Sensor fluid 20 may be a substantially electrolyte-free liquid such as pharmaceutical-grade purified water (USP grade water). In some aspects of the disclosure distilled water may also be used.

Winding filament 22 is used to secure the membrane body 18 to sensor body 12. Adhesive may be used to bond and reinforce the winding filament 22.

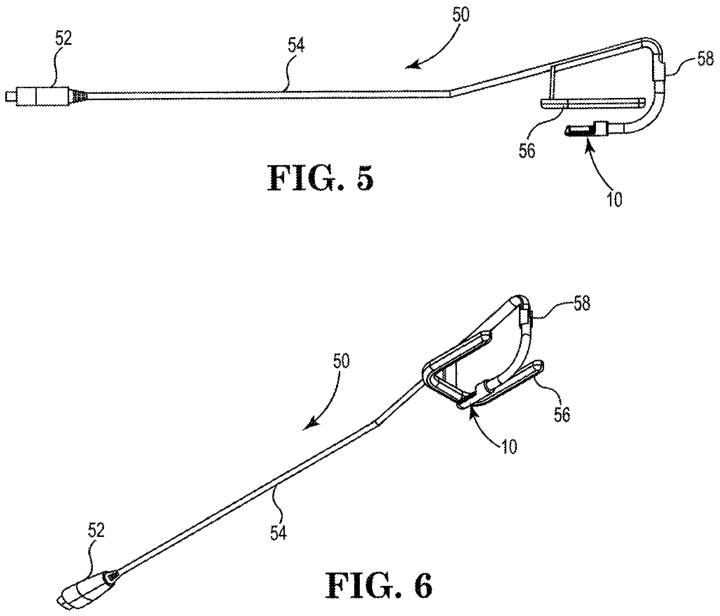

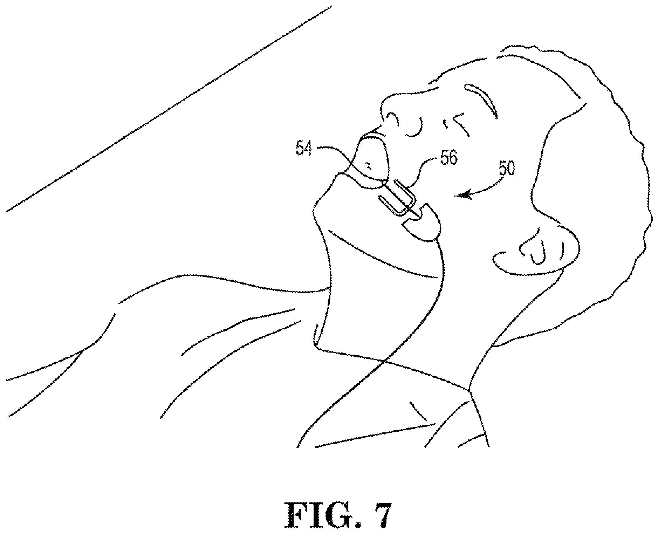

Referring now to FIGS. 5 - 7 a sensor placement device 50 for secure placement of the sensor 10 against a buccal surface is illustrated. The sensor placement device broadly includes proximal end 52, elongate middle portion 54 and distal end 56. Proximal end 52 is adapted to operably couple to electronics for reading and displaying the pCO₂ measurements. Distal end 56 includes U-shaped ridge 56 for positioning the device 50 against the outside surface of the cheek and the sensor on the inside of the cheek. Sensor 10 is attached to the positioning device 50 by arm 58.

In operation to be used to measure tissue pCO₂ in the oral cavity, the sensor 10 is deployed in a sensor placement device 50 configured to fit the human cheek. As shown, sensor placement device 50 with sensor 10 is a disposable device. Using the elongate middle portion, a user may insert the sensor 10 into the mouth of a subject and position a U-shaped ridge at the distal end 56 to the outside surface of the cheek such that the cheek of the subject is positioned between the arm 58 that includes the sensor 10 and the U-shaped ridge of the distal end 56. This, in turn, holds the sensor 10 against the buccal surface of the cheek and between the two arms of the U-shaped ridge such that the two arms of the U-shaped ridge fold the buccal tissue (i.e., the inside of the cheek) against the membrane (e.g., cylindrical, dome-shaped, etc.) of the sensor 10 for providing optimal contact without excessive pressure application. The device 50 is designed (i.e., the spacing between the sensor plane and the U-shaped ridge is configured) so the sensor 10 is held in direct contact with the buccal tissue without air gaps and without applying pressure in excess of 25 mm Hg and preferably less than 20 mm Hg, less than 15 mm Hg, or the like. Excess pressure can disturb blood flow and alter the level of pCO₂. A person of skill in the art will understand that the U-shaped ridge may be any suitable shape such as a, a V-shape, a c-shape, a square loop-shape, a triangular loop shape, an oval loop shape, or the like (however only the U-shape, V-shape, or C-shape are part of the claimed invention and not the square loop-shape, the triangular loop shape and the oval loop shape).

The response time of the sensor 10 for measuring the pCO₂ may be influenced by the ratio of surface area (that allows analyte to pass through) to the volume of the sensor. If the sensor is positioned next to the mucosal surface (no pressure applied), a cylindrical shaped membrane of sensor will have only a small percentage of the membrane directly in contact with the tissue (tangential) leading to an increase in response time. If suitable pressure is applied to push the cylindrical surface into the tissue, about 40 to 50% of the membrane surface may contact the tissue as the surface is deflected away from the pressure. However, the applied pressure must be carefully modulated so as not to disturb microcapillary blood flow and introduce an error in the measurements. The sensor placement device 50 of the current disclosure is configured such that it folds the buccal tissue about the cylindrical membrane surface of the sensor 10 to achieve this greater tissue contact without applying excessive pressure. Optionally, the sensor cover may also be tapered away from the membrane surface to allow for the higher percentage of tissue contact.

Sensor cabling (not shown) attaches the sensor placement device with sensor to electronic equipment (not shown) that provides an alternating electrical potential to the sensor 10 and measures the impedance of the sensor fluid 20 contained within membrane body 18. The equipment is calibrated to the sensor response curve and an algorithm calculates the pCO₂ value from the temperature-adjusted conductance signal. The sensor response curve is determined by measuring the sensor signal in two reference solutions of known pCO₂ levels; a low pCO₂ reference and a "normal" pCO₂ reference. The "normal" solution approximates the pCO₂ of healthy, well perfused tissue. From this data, the slope of the response curve is determined. Values of pCO₂ are then calculated from the signal difference from the "normal" reference solution. The calculated pCO₂ values are then displayed graphically and numerically on an integrated display. The electronic device is configured as a standalone patient monitoring device, but those of skill in the art will appreciate that it can be integrated into a multi-modal patient monitoring system.

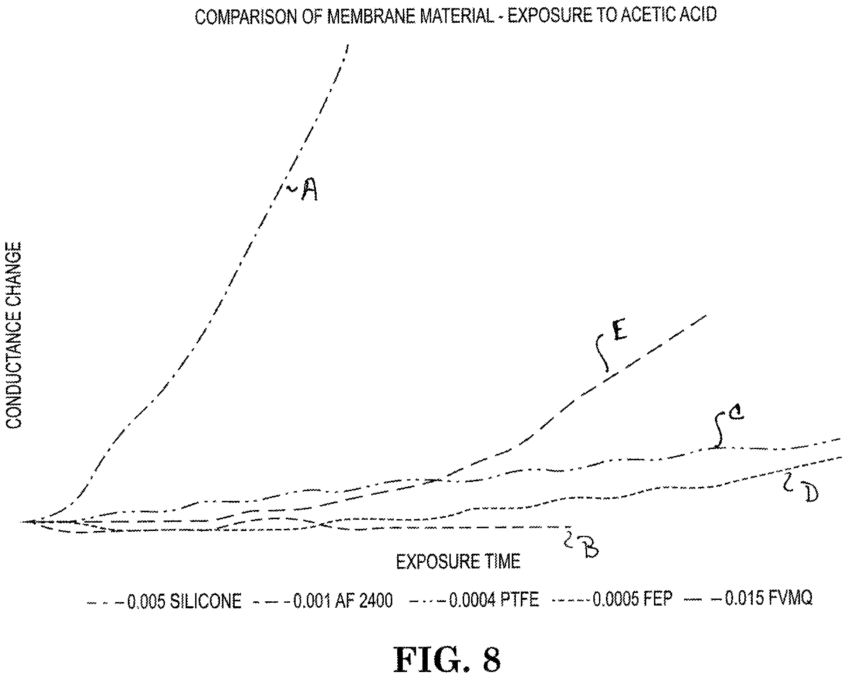

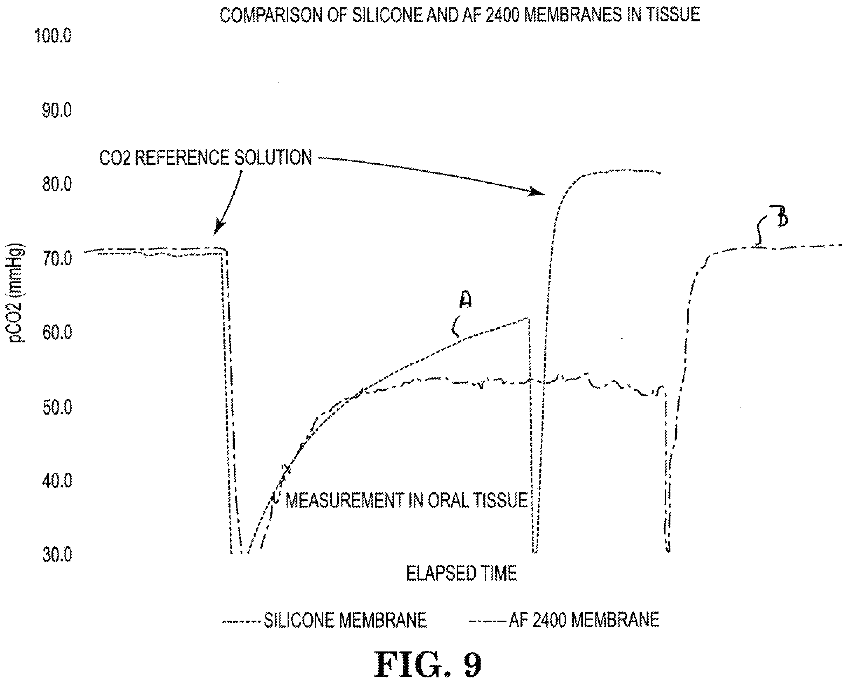

Referring now to FIGS. 8 and 9 comparison data will now be discussed. FIGS. 8 graphically illustrate the results of an *in vitro* study of several membrane materials. These membranes were exposed to 8mM acetic acid solution and the conductance change was monitored in an effort to determine suitability for application in the oral cavity. Membrane thicknesses were chosen based on the ability to achieve a reasonable carbon dioxide permeation rates. The results demonstrate the superiority of the Teflon AF 2400. Fig 8 shows a comparison of potential membrane materials evaluated at thicknesses necessary to provide comparable response times wherein A - 0.005" PDMS Silicone; B - 0.001" Teflon AF2400; C - 0.0004" PTFE; D - 0.0005" FEP; and E - 0.015" FVMQ Silicone.

A conductance probe was cover with the material being tested and then exposed to 8mM Acetic acid (in the physiological range for saliva). Of the materials tested, the typical membrane material (A - PDMS Silicone) is the most permeable to acetic acid. In 60 minutes, the conductance has increased by 1uS/cm due to acetic acid crossing PDMS silicone membrane. Membrane E (0.015" FVMQ Silicone) allowed an increase of 0.12uS/cm during that time while Membrane C (0.0004" PTFE) and membrane D (0.0005" FEP) showed better resistance to acetic acid penetration, ~0.05 uS/cm over 60 minutes. However, membrane B (0.001" Teflon AF2400) allowed no detectable increase in conductance over that same time period.

FIG. 9 graphically illustrates an overlay of in vivo studies of a sensor constructed with a silicone membrane compared to a sensor constructed with a Teflon AF 2400 membrane. A reference solution was measured pre- and post-exposure to oral mucosal tissue. The results demonstrate the contamination that can occur with the use of a silicone membrane, as well as demonstrating the suitability of Teflon AF 2400. FIG. 9 depicts a comparison of tissue data collected with a PDMS silicone membrane (A) and a Teflon AF2400 membrane (B). Reference values were measured in a water tonometered with 10% CO2 (pCO2 = ~70mmHg). Sensors were then positioned into a subjects buccal tissue and data was collected for about 60 minutes. The graph indicates that the Teflon AF2400 membrane sensor stabilized at ~53mmHg pCO2, while the PDMS silicone membrane sensor continued to rise past 60mmHg pCO2 and never stabilized. Sensors were placed back into the tonometers. The Teflon AF2400 membrane sensor returned to the pre-tissue exposure value while the Silicone membrane sensor indicates an error of ~10mmHg pCO2 correlating to increased signal caused by the acetic acid contamination.

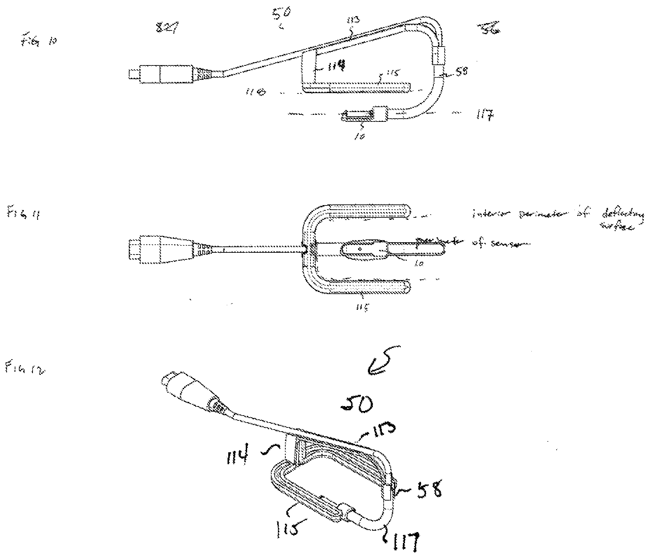

Referring now to FIG. 10 a side view of the sensor placement device 50 in accordance with the disclosure is shown. The device 50 positions sensor 10 against tissue. The sensor placement device 50 broadly includes curved sensor arm 58, angled beam 113, connecting post 114, and deflecting surface 115. The sensor placement device 50 provides ample space so as not to squeeze the tissue against which it is positioning the sensor 10. Rather, the sensor placement device 50 (e.g., the two arms of the U-shaped portion) utilizes the flexibility of the buccal tissue to fold the tissue around the cylindrical membrane of the sensor 10 to achieve 40-50% or greater contact with the tissue, as hereinafter disclosed. The device 50 includes a sensor arm 58 on one plane and two arms equidistant distant from the sensor arm 58 that together form the U-shaped deflecting surface 115 on a separate plane such that the sensor 10 is positioned between the two arms of the U-shaped deflecting surface 115, as best seen in FIG 11. This way the deformability of the device 50 material is not required.

Sensor 10 measures an analyte or characteristic indicative of microcirculatory blood flow. The membrane of the sensor 10 is preferably cylindrical or domed or has a suitable shape characteristic that adapts to having tissue folded over it. If the sensor 10 measures a gas, the sensor 10 requires a sensor cover 12 to protect the sensor from exposure to ambient and end-tidal gases. Sensor arm 58 may be constructed of a thermoplastic such as engineered thermoplastic polyurethane, polyethylene, polypropylene, polystyrene and polycarbonate. Sensor arm 58 attaches to beam 113 that in turn attaches to the top of post 114. In this manner, sensor arm 58 is configured to hold sensor 10 on the sensor plane 117. Beam 113 and post 114 may also be constructed of a thermoplastic such as engineered thermoplastic polyurethane, polyethylene, polypropylene, polystyrene and polycarbonate.

Deflecting surface 115, similarly constructed of a thermoplastic such as engineered thermoplastic polyurethane, polyethylene, polypropylene, polystyrene and polycarbonate, is attached to the bottom of post 114. In this manner the bottom of deflecting surface 115 defines deflecting surface plane 118. To ensure contact on the low end of normal buccal tissue thickness (approximately 7 mm), sensor arm plane 117 and deflecting surface plane 118 may be less than about 5 mm apart, about 4 - 6 mm apart, about 5 mm apart, about 3-5 mm apart, or the like such that the pressure exerted does not exceed about 25 mm Hg that can cause occlusion of blood flow and errors in the measured pCO₂. The line of sight above sensor 10 is ideally free of obstructions for at least 20 mm, at least about 10 mm, at least about 15 mm, about 15-25 mm, or the like to prevent pinching of tissue between surfaces of the device 50. Optionally, to adapt to thicker buccal tissue, the interior perimeter of deflecting surface 115 may be offset from the perimeter of sensor 10 (as shown in FIG. 11) by about 15 mm to about 20 mm, about 16 mm to about 19 mm, about 17 mm to about 18 mm, about 15 mm to about 20 mm, or the like.

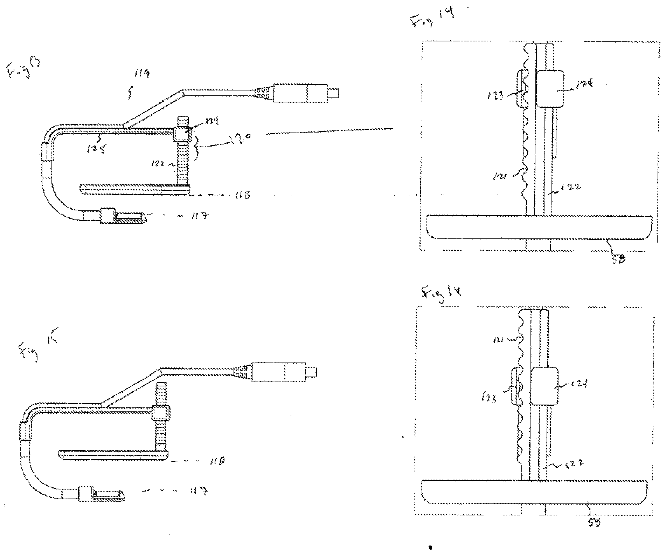

FIG. 13 illustrates a sensor placement device 119 that is similar to that disclosed in FIG. 10; however includes a ratcheting element 120 configured for modifying and controlling the separation between the sensor plane 117 and deflecting surface plane 118. Ratcheting element 120 is shown in partial view FIG. 14 corresponding to a separation of about 5 mm between sensor plane 117 and deflecting surface plane 118. Ratcheting element 120 may include serrations 121 found on post 122 and complementary ratchet tooth 123 on beam head 124. Beam head 124 is further attached to beam 125 and has alignment features that mate with post 122. The beam head may move up and down the post 122 via engagement of the serrations 121 with the complementary ratchet tooth 123. FIG. 15 and FIG. 16 illustrate an increase in the separation between sensor plane 117 and deflecting surface plane 118 via ratcheting feature 120. In this manner, device 119 may be adaptable to a wide range of buccal tissue thickness.

## Claims

1. A sensor system for measuring partial pressure of carbon dioxide, pCO₂, in tissue, the sensor system comprising:
a sensor (10) comprising:
a sensor cover (12) forming an opening (26) housing a sensor body (14) and a membrane body (18), the sensor cover (12) including a lip (24) extending downwardly from a distal end of the sensor cover (12), the opening being bounded on a top by the underside of the sensor cover (12);
the membrane body (18) comprising a first amorphous fluoroplastic, the membrane body (18) forming an enclosed chamber including:
a first end (28), and
a second closed end (30) situated against the lip (24);
the sensor body (14) disposed at the first end (28) of the membrane body (18) for coupling the first end (28) of the membrane body (18) to the sensor cover (12);
two or more electrodes (16) extending from the first end (28) of the membrane body (18) and positioned within the enclosed chamber; and
a substantially electrolyte-free liquid (20) contained within the enclosed chamber and in contact with the two or more electrodes (16),
wherein the lip (24) is configured to shield the membrane body (18) from end tidal carbon dioxide when the sensor (10) is in use, and the sensor cover (12) is designed to cover the portion of the membrane body (18) that is not in contact with tissue; and
a sensor placement device (50) configured to facilitate placement of the sensor (10) against tissue of a subject, the sensor placement device (50) comprising a sensor arm (58), a deflecting surface (115) and a beam (113) coupling the sensor arm (58) to the deflecting surface (115), wherein:
a first end of the sensor arm (58) is configured to couple with the sensor (10) such that the sensor is in a sensor plane (117);
the deflecting surface (115) lies in a deflecting surface plane (118) that is offset from the sensor plane (117);
wherein the deflecting surface (115) comprising a pair of arms forming a U-shape, V-shape, or C-shape configured to fold buccal tissue over the membrane body (18) of the sensor (10);
and the beam (113) comprises a ratcheting element (120) configured to change the offset between the sensor plane (117) and the deflecting surface plane (118).

2. A sensor system of claim 1, wherein the offset between the sensor plane (117) and the deflecting surface plane (118) is at least 5 mm.

3. The sensor system of claim 2, wherein the membrane body (18) is cylindrical or spherical.

4. The sensor system of claim 2, wherein the deflecting surface (115) comprises a U-shaped portion formed by two arms that are equidistant to the sensor (10) coupled to the first end of the sensor arm (58).

5. A sensor system of claim 4, wherein the sensor placement device (50) is configured to place the sensor (10) directly against the buccal tissue while applying pressure of 25 mm Hg or less by folding the buccal tissue over the membrane body (18) via the two arms of the deflecting surface (115).

6. The sensor system of claim 1, wherein the ratcheting element (120) comprises:
a post (122) comprising serrations (121),
a beam head (124) comprising a ratchet tooth (123) complementary to the serrations (121),
wherein the beam head (124) is configured to move in two opposing directions along the post (122) via engagement of the serrations (121) with the complementary tooth (123) so as to change the offset between the sensor plane (117) and the deflecting surface plane (118).

7. The sensor system of claim 1, wherein the sensor (10) is responsive to an alternating electrical potential to measure the impedance of the substantially electrolyte-free liquid (20).

8. A method for determining partial pressure of carbon dioxide, pCO₂, in tissue, the method comprising:
providing a sensor (10) comprising a sensor cover (12) forming an opening (26) housing a sensor body (14) and a membrane body (18), the sensor cover (12) including a lip (24) extending downwardly from a distal end of the sensor cover (12), the opening being bounded on a top by the underside of the sensor cover (12),
the membrane body (18) comprising a first amorphous fluoroplastic, the membrane body (18) forming an enclosed chamber including:
a first end (28), and
a second closed end (30) situated against the lip (24) and the sensor body (14) disposed at the first end (28) of the membrane body (18) for coupling the first end (28) of the membrane body (18) to the sensor cover (12)
wherein two or more electrodes (16) extend from the first end (28) of the membrane body (18) and positioned within the enclosed chamber,
wherein a substantially electrolyte-free liquid (20) is contained within the enclosed chamber and in contact with the two or more electrodes (16), and
wherein the lip (24) is configured to shield the membrane body (18) from end tidal carbon dioxide when the sensor (10) is in use, and the sensor cover (12) is designed to cover the portion of the membrane body (18) that is not in contact with tissue,
placing, using a sensor placement device (50), the sensor (10) proximate to and in direct contact with a buccal tissue of a subject while pressure is applied that is equal to or less than 25 mm Hg,
the sensor placement device (50) comprising a sensor arm (58), a deflecting surface (115) and a beam (113) coupling the sensor arm (58) to the deflecting surface (115), wherein:
a first end of the sensor arm (58) is configured to couple with the sensor (10) such that the sensor is in a sensor plane (117);
the deflecting surface (115) lies in a deflecting surface plane (118) that is offset from the sensor plane (117);
wherein the deflecting surface (115) comprising a pair of arms forming a U-shape, V-shape, or C-shape configured to fold buccal tissue over the membrane body (18) of the sensor (10);
using a ratcheting element (120) of the beam (113) to change the offset between the sensor plane (117) and the deflecting surface plane (118); and
measuring, using the sensor (10), the pCO₂ in the buccal tissue;
wherein the sensor cover (12) is designed to cover the portion of the membrane body (18) that is not in contact with tissue.

9. The method of claim 8, further comprising controlling the offset between the sensor plane (117) and the deflecting surface plane (118) to be 5 mm.

## Patentansprüche

1. Sensorsystem zum Messen von Kohlendioxidpartialdruck, pCO₂, in Gewebe, wobei das Sensorsystem Folgendes beinhaltet:
einen Sensor (10), der Folgendes beinhaltet:
eine Sensorabdeckung (12), die eine Öffnung (26) bildet, in der ein Sensorkörper (14) und ein Membrankörper (18) aufgenommen sind, wobei die Sensorabdeckung (12) eine Lippe (24) aufweist, die sich von einem distalen Ende der Sensorabdeckung (12) nach unten erstreckt, wobei die Öffnung an einem oberen Ende durch die Unterseite der Sensorabdeckung (12) begrenzt ist;
den Membrankörper (18), der einen ersten amorphen Fluorkunststoff beinhaltet, wobei der Membrankörper (18) eine umschlossene Kammer bildet, die Folgendes aufweist:
ein erstes Ende (28) und
ein zweites geschlossenes Ende (30), das an der Lippe (24) liegt;
den Sensorkörper (14), der an dem ersten Ende (28) des Membrankörpers (18) angeordnet ist, um das erste Ende (28) des Membrankörpers (18) an die Sensorabdeckung (12) anzukoppeln;
zwei oder mehr Elektroden (16), die sich von dem ersten Ende (28) des Membrankörpers (18) erstrecken und innerhalb der umschlossenen Kammer positioniert sind; und
eine im Wesentlichen elektrolytfreie Flüssigkeit (20), die in der umschlossenen Kammer enthalten ist und mit den zwei oder mehr Elektroden (16) in Kontakt ist,
wobei die Lippe (24) für das Abschirmen des Membrankörpers (18) gegen endtidales Kohlendioxid ausgelegt ist, wenn der Sensor (10) in Gebrauch ist, und die Sensorabdeckung (12) für das Abdecken des Teils des Membrankörpers (18) ausgelegt ist, der nicht mit Gewebe in Kontakt ist; und
eine Sensoransetzvorrichtung (50), die für das Ermöglichen des Ansetzens des Sensors (10) an Gewebe einer Zielperson ausgelegt ist, wobei die Sensoransetzvorrichtung (50) einen Sensorarm (58), eine Ablenkfläche (115) und einen Schaft (113), der den Sensorarm (58) an die Ablenkfläche (115) ankoppelt, beinhaltet, wobei:
ein erstes Ende des Sensorarms (58) für das Ankoppeln an den Sensor (10) ausgelegt ist, so dass der Sensor sich in einer Sensorebene (117) befindet;
die Ablenkfläche (115) in einer Ablenkflächenebene (118) liegt, die von der Sensorebene (117) versetzt ist;
wobei die Ablenkfläche (115) ein Paar Arme beinhaltet, die eine U-Form, eine V-Form oder eine C-Form bilden, die dafür ausgelegt ist, bukkales Gewebe über den Membrankörper (18) des Sensors (10) zu falten; und
der Schaft (113) ein Gesperreelement (120) beinhaltet, das dafür ausgelegt ist, den Versatz zwischen der Sensorebene (117) und der Ablenkflächenebene (118) zu ändern.

2. Sensorsystem nach Anspruch 1, wobei der Versatz zwischen der Sensorebene (117) und der Ablenkflächenebene (118) mindestens 5 mm beträgt.

3. Sensorsystem nach Anspruch 2, wobei der Membrankörper (18) zylindrisch oder kugelförmig ist.

4. Sensorsystem nach Anspruch 2, wobei die Ablenkfläche (115) einen U-förmigen Teil beinhaltet, der von zwei Armen gebildet wird, die in gleichem Abstand zu dem Sensor (10) sind, der an den ersten Ende des Sensorarms (58) angekoppelt ist.

5. Sensorsystem nach Anspruch 4, wobei die Sensoransetzvorrichtung (50) zum Ansetzen des Sensors (10) direkt an das bukkale Gewebe unter Ausüben eines Drucks von 25 mm Hg oder weniger durch Falten des bukkalen Gewebes über den Membrankörper (18) durch die zwei Arme der Ablenkfläche (115) vorgesehen ist.

6. Sensorsystem nach Anspruch 1, wobei das Gesperreelement (120) Folgendes beinhaltet:
eine Säule (122), die eine Verzahnung (121) beinhaltet,
einen Schaftkopf (124), der einen zur Verzahnung (121) passenden Sperrzahn (123) beinhaltet,
wobei der Schaftkopf (124) dafür ausgelegt ist, sich über den Eingriff der Verzahnung (121) mit dem dazu passenden Zahn (123) in zwei entgegengesetzten Richtungen am Schaft (122) entlangzubewegen, um den Versatz zwischen der Sensorebene (117) und der Ablenkflächenebene (118) zu ändern.

7. Sensorsystem nach Anspruch 1, wobei der Sensor (10) zum Messen der Impedanz der im Wesentlichen elektrolytfreien Flüssigkeit (20) auf ein wechselndes elektrisches Potenzial reagiert.

8. Verfahren zum Bestimmen von Kohlendioxidpartialdruck, pCO₂, in Gewebe, wobei das Verfahren Folgendes beinhaltet:
Bereitstellen eines Sensors (10), der Folgendes beinhaltet: eine Sensorabdeckung (12), die eine Öffnung (26) bildet, in der ein Sensorkörper (14) und ein Membrankörper (18) aufgenommen sind, wobei die Sensorabdeckung (12) eine Lippe (24) aufweist, die sich von einem distalen Ende der Sensorabdeckung (12) nach unten erstreckt, wobei die Öffnung an einem oberen Ende durch die Unterseite der Sensorabdeckung (12) begrenzt ist,
den Membrankörper (18), der einen ersten amorphen Fluorkunststoff beinhaltet, wobei der Membrankörper (18) eine umschlossene Kammer bildet, die Folgendes aufweist:
ein erstes Ende (28) und
ein zweites geschlossenes Ende (30), das an der Lippe (24) liegt, und den Sensorkörper (14), der an dem ersten Ende (28) des Membrankörpers (18) angeordnet ist, um das erste Ende (28) des Membrankörpers (18) an die Sensorabdeckung (12) anzukoppeln;
wobei sich zwei oder mehr Elektroden (16) von dem ersten Ende (28) des Membrankörpers (18) erstrecken und innerhalb der umschlossenen Kammer positioniert sind,
wobei eine im Wesentlichen elektrolytfreie Flüssigkeit (20) in der umschlossenen Kammer enthalten ist und mit den zwei oder mehr Elektroden (16) in Kontakt ist, und
wobei die Lippe (24) für das Abschirmen des Membrankörpers (18) gegen endtidales Kohlendioxid ausgelegt ist, wenn der Sensor (10) in Gebrauch ist, und die Sensorabdeckung (12) für das Abdecken des Teils des Membrankörpers (18) ausgelegt ist, der nicht mit Gewebe in Kontakt ist;
Ansetzen, unter Verwendung einer Sensoransetzvorrichtung (50), des Sensors (10) nahe an und in direktem Kontakt mit einem bukkalen Gewebe einer Zielperson, während Druck ausgeübt wird, der 25 mm Hg oder weniger beträgt,
wobei die Sensoransetzvorrichtung (50) einen Sensorarm (58), eine Ablenkfläche (115) und einen Schaft (113), der den Sensorarm (58) an die Ablenkfläche (115) ankoppelt, beinhaltet, wobei:
ein erstes Ende des Sensorarms (58) für das Ankoppeln an den Sensor (10) ausgelegt ist, so dass der Sensor sich in einer Sensorebene (117) befindet;
die Ablenkfläche (115) in einer Ablenkflächenebene (118) liegt, die von der Sensorebene (117) versetzt ist;
wobei die Ablenkfläche (115) ein Paar Arme beinhaltet, die eine U-Form, eine V-Form oder eine C-Form bilden, die dafür ausgelegt ist, bukkales Gewebe über den Membrankörper (18) des Sensors (10) zu falten;
Verwenden eines Gesperreelements (120) des Schafts (113) um den Versatz zwischen der Sensorebene (117) und der Ablenkflächenebene (118) zu ändern; und
Messen, unter Verwendung des Sensors (10), des pCO₂ in dem bukkalen Gewebe;
wobei die Sensorabdeckung (12) für das Abdecken des Teils des Membrankörpers (18) ausgelegt ist, der nicht mit Gewebe in Kontakt ist.

9. Verfahren nach Anspruch 8, das ferner das Regeln des Versatzes zwischen der Sensorebene (117) und der Ablenkflächenebene (118) auf 5 mm beinhaltet.

## Revendications

1. Système de capteur pour mesurer la pression partielle de dioxyde de carbone, pCO₂, dans un tissu, le système de capteur comprenant :
un capteur (10) comprenant :
un couvercle de capteur (12) formant une ouverture (26) logeant un corps de capteur (14) et un corps membranaire (18), le couvercle de capteur (12) comprenant une lèvre (24) s'étendant vers le bas d'une extrémité distale du couvercle de capteur (12), l'ouverture étant délimitée sur un dessus par le dessous du couvercle de capteur (12) ;
le corps membranaire (18) comprenant un premier fluoroplastique amorphe, le corps membranaire (18) formant une enceinte fermée comprenant :
une première extrémité (28) ; et
une deuxième extrémité fermée (30) située contre la lèvre (24) ;
le corps de capteur (14) étant placé à la première extrémité (28) du corps membranaire (18) pour coupler la première extrémité (28) du corps membranaire (18) au couvercle de capteur (12) ;
deux électrodes ou plus (16) s'étendant de la première extrémité (28) du corps membranaire (18) et placées dans l'enceinte fermée ; et
un liquide sensiblement dépourvu d'électrolyte (20) contenu dans l'enceinte fermée et en contact avec les deux électrodes (16) ou plus,
dans lequel la lèvre (24) est configurée pour protéger le corps membranaire (18) du dioxyde de carbone en fin d'expiration lorsque le capteur (10) est utilisé, et le couvercle de capteur (12) est conçu pour couvrir la partie du corps membranaire (18) qui n'est pas en contact avec le tissu ; et
un dispositif de mise en place de capteur (50) configuré pour faciliter la mise en place du capteur (10) contre un tissu d'un sujet, le dispositif de mise en place de capteur (50) comprenant un bras de capteur (58), une surface de déflexion (115) et une tige (113) couplant le bras de capteur (58) à la surface de déflexion (115), dans lequel :
une première extrémité du bras de capteur (58) est configurée pour se coupler au capteur (10) de sorte que le capteur est dans un plan de capteur (117) ;
la surface de déflexion (115) se trouve dans un plan de surface de déflexion (118) qui est décalé du plan de capteur (117) ;
dans lequel la surface de déflexion (115) comprend une paire de bras formant une forme en U, une forme en V, ou une forme en C, configurés pour tenir le tissu buccal sur le corps membranaire (18) du capteur (10) ; et
la tige (113) comprend un élément à cliquet (120) configuré pour changer le décalage entre le plan de capteur (117) et le plan de la surface de déflexion (118).

2. Système de capteur selon la revendication 1, dans lequel le décalage entre le plan de capteur (1170) et le plan de la surface de déflexion (118) est d'au moins 5 mm.

3. Système de capteur selon la revendication 2, dans lequel le corps membranaire (18) est cylindrique ou sphérique.

4. Système de capteur selon la revendication 2, dans lequel la surface de déflexion (115) comprend une partie en forme de U formée par deux bras qui sont équidistants au capteur (10) couplés à la première extrémité du bras de capteur (58).

5. Système de capteur selon la revendication 4, dans lequel le dispositif de mise en place de capteur (50) est configuré pour placer le capteur (10) directement contre le tissu buccal tout en appliquant une pression de 25 mmHg ou moins en pliant le tissu buccal sur le corps membranaire (18) par les deux bras de la surface de déflexion (115).

6. Système de capteur selon la revendication 1, dans lequel l'élément à cliquet (120) comprend :
un montant (122) comprenant des crans (121),
une tête de tige (124) comprenant une dent de cliquet (123) complémentaire aux crans (121),
dans lequel la tête de tige (124) est configurée pour se déplacer dans deux directions opposées le long du montant (122) par l'engagement des crans (121) avec la dent complémentaire (123) de manière à changer le décalage entre le plan de capteur (117) et le plan de la surface de déflexion (118).

7. Système de capteur selon la revendication 1, dans lequel le capteur (10) est sensible à un potentiel électrique alternatif pour mesurer l'impédance du liquide sensiblement dépourvu d'électrolyte (20).

8. Procédé de détermination de pression partielle de dioxyde de carbone, pCO₂, dans un tissu, le procédé comprenant :
fournir un capteur (10) comprenant un couvercle de capteur (12) formant une ouverture (26) logeant un corps de capteur (14) et un corps membranaire (18), le couvercle de capteur (12) comprenant une lèvre (24) s'étendant vers le bas de l'extrémité distale du couvercle de capteur (12), l'ouverture étant délimitée sur le haut par le dessous du couvercle de capteur (12),
le corps membranaire (18) comprenant un premier fluoroplastique amorphe, le corps membranaire (18) formant une enceinte fermée comprenant :
une première extrémité (28), et
une deuxième extrémité fermée (30) située contre la lèvre (24) et le corps de capteur (14) étant placé à la première extrémité (28) du corps membranaire (18) pour coupler la première extrémité (28) du corps membranaire (18) au couvercle de capteur (12) ;
dans lequel deux électrodes ou plus (16) s'étendent de la première extrémité (28) du corps membranaire (18) et sont placées dans l'enceinte fermée,
dans lequel le liquide sensiblement dépourvu d'électrolyte (20) est contenu dans l'enceinte fermée et est en contact avec les deux électrodes ou plus (16), et
dans lequel la lèvre (24) est configurée pour protéger le corps membranaire (18) du dioxyde de carbone en fin d'expiration lorsque le capteur (10) est utilisé, et le couvercle de capteur (12) est conçu pour couvrir la partie du corps membranaire (18) qui n'est pas en contact avec le tissu ;
placer le capteur près de et en contact direct avec un tissu buccal d'un sujet tout en appliquant une pression égale ou inférieure à 25 mmHg, en utilisant le dispositif de mise en place de capteur (50),
le dispositif de mise en place de capteur (50) comprenant un bras de capteur (58), une surface de déflexion (115) et une tige (113) couplant le bras de capteur (58) à la surface de déflexion (115), dans lequel :
une première extrémité du bras de capteur (58) est configurée pour se coupler au capteur (10) de sorte que le capteur est dans un plan de capteur (117) ;
la surface de déflexion (117) se trouve dans un plan de surface de déflexion (118) qui est décalé du plan de capteur (117) ;
dans lequel la surface de déflexion (115) comprend une paire de bras formant une forme en U, une forme en V ou une forme en C, configurés pour tenir le tissu buccal sur le corps membranaire (18) du capteur (10) ;
utiliser un élément à cliquet (120) de la tige (113) pour changer le décalage entre le plan de capteur (117) et le plan de la surface de déflexion (118) ; et
mesurer la pCO₂ dans le tissu buccal en utilisant le capteur (10) ;
dans lequel le couvercle de capteur (12) est conçu pour couvrir la partie du corps membranaire (18) qui n'est pas en contact avec le tissu.

9. Procédé selon la revendication 8, comprenant en outre contrôler le décalage entre le plan de capteur (117) et le plan de la surface de déflexion (118).
